# EUROPEAN PATENT APPLICATION

(11) **EP 4 685 549 A1**
(43) Date of publication of application: **28.01.2026**
(21) Application number: 24774599.5
(22) Date of filing: 27.02.2024
(51) Int. Cl.: G02C 13/00, A61B 3/028, G02C 7/06

(54) **METHOD FOR DETERMINING PROGRESSIVE REFRACTIVE POWER LENS**

(30) Priority: 20.03.2023 JP 2023043950
(71) Applicant: Hoya Lens Thailand Ltd., Pathumthani 12130 (TH)
(72) Inventor: MATSUOKA Shohei, Tokyo 160-8347 (JP); KAGA Tadashi, Tokyo 160-8347 (JP); ITO Ayumu, Tokyo 160-8347 (JP); SONODA Yutaro, Tokyo 160-8347 (JP)
(74) Representative: Betten & Resch
(86) International application number: PCT/JP2024/007184
(87) International publication number: WO 2024/195454

(57) **Abstract**

There is provided a method for determining a progressive power lens, the method including: determining a necessity for expanding a clear vision area for a subject wearing a progressive power lens; and when it is determined that the subject requires expansion of the clear vision area in a depth direction, selecting a design of the progressive power lens in which the clear vision area is expanded in the depth direction by reducing an addition power in an intermediate portion of a progressive zone of the progressive power lens.

## Description

### Technical field

The present invention relates to a method for determining a progressive power lens.

### Description of related art

Patent document 1 and patent document 2 disclose a progressive power lens having a distance portion, a near portion and an intermediate portion where transmission astigmatism is added to the near and intermediate portions, and the near and intermediate portions to which transmission astigmatism is added include portions where a refractive power has been subtracted for correcting astigmatism in such a manner that an amount of horizontal refractive power is larger than an amount of vertical refractive power (or, an amount of vertical refractive power is larger than an amount of horizontal refractive power).

Also, patent document 3 discloses a spectacle prescription assistance device including an obtaining means for obtaining first distribution data, which is measurement data of the test eye obtained by a wavefront sensor, regarding a distribution of refractive error in a test eye, and second distribution data, which is measurement data for a part of the measurement data on the refractive power distribution of the spectacle lens for correcting the refractive error of the subject's eye and measured by a lens meter; and a calculation means for obtaining third distribution data regarding a distribution of refractive error taking into account correction by the spectacle lens, based on the first distribution data and the second distribution data obtained by the obtaining means.

Also, patent document 4 discloses a method for determining a human visual behavior, the method including: recording a head movement of a person while performing a visual task; recording at least one eyeball movement of the person while performing the visual task; determining an orientation of the eyeball relative to a head at different times; and determining an amount of time the eyeballs were held in each orientation.

Further, non-patent document 1 suggests that optimizing vision within a standard arm length range increases comfort for a wearer. As a means for achieving this, patent document 5 discloses a method for evaluating a clear vision area in a Cartesian coordinate system, and patent document 6 discloses a method for adjusting an addition curve to suit an individual.

### Prior art document

### Patent document

[Patent Document 1] International Publication No. 2020/067522
[Patent Document 2] International Publication No. 2020/067523
[Patent Document 3] Japanese Patent No. 6708955
[Patent Document 4] Japanese Patent No. 4659027
[Patent Document 5] Patent No. 6920287
[Patent Document 6] Patent No. 6991135

### Non-patent Document

[Non-patent Document 1] Varilux X series, searched on March 9, 2023, Internet < URL:https://www.essilor.sa/en/products/varilux-x-series> <URL: https://www.cssilor.sa/en/products/varilux-x-series>

### Summary of the invention

### Problem to be solved by the invention

An object of one embodiment of the present invention is to provide a technique capable of determining a progressive power lens that can be worn more comfortably by a subject who requires expansion of a clear vision area in a depth direction.

### Means for solving the problem

According to a first aspect of the present invention, there is provided a method for determining a progressive power lens, the method including:
determining a necessity for expanding a clear vision area for a subject wearing a progressive power lens; and when it is determined that the subject requires expansion of the clear vision area in a depth direction,
selecting a design of the progressive power lens in which the clear vision area is expanded in the depth direction by reducing an addition power in an intermediate portion of a progressive zone of the progressive power lens.

According to a second aspect of the present invention, there is provided the method for determining a progressive power lens according to the first aspect, wherein in the determination step, the necessity for expanding the clear vision area is determined based on a transition of a line of sight movement of the subject when the subject alternates between near vision and far vision.

According to a third aspect of the present invention, there is provided the method for determining a progressive power lens according to the first aspect, wherein in the determination step, the necessity for expanding the clear vision area is determined based on an amount of third-order aberration in the horizontal direction of the subject's eye.

According to a fourth aspect of the present invention, there is provided the method for determining a progressive power lens according to the first aspect, wherein in the determination step, the necessity for expanding the clear vision area is determined based on a transition movement of an object when the subject performs a task including actions of holding, viewing, and moving the object whose position information can be acquired.

According to a fifth aspect of the present invention, there is provided the method for determining a progressive power lens according to the first aspect, wherein in the design selection step, when the subject's eye is an origin, a vertical direction is Y axis, and the depth direction is Z axis, a design with reduced addition power is selected so that a position of Y = - 210 mm, Z = 850 mm is within the clear vision area.

According to a sixth aspect of the present invention, there is provided the method for determining a progressive power lens according to the first aspect, wherein in the design selection step, a design of the progressive power lens is selected in which the clear vision area is expanded in the depth direction in exchange for increasing an amount of astigmatism on a principal meridian of the progressive power lens.

According to a seventh aspect of the present invention, there is provided the method for determining a progressive power lens according to the first aspect, wherein in the design selection step, by reducing the addition power, a design of a progressive power lens design is selected in which the clear vision area is expanded in the depth direction and horizontal direction.

According to an eighth aspect of the present invention, there is provided the method for determining a progressive power lens according to the first aspect, wherein when it is determined that the subject requires expansion of the clear vision area in a horizontal direction in the determination step, a design of the progressive power lens is selected in the design selection step in which a power distribution of the progressive power lens is smoothed in the horizontal direction to reduce third-order aberration in the horizontal direction around a principal meridian of the progressive power lens, thereby expanding the clear vision area in the horizontal direction.

According to a nineth aspect of the present invention, there is provided the method for determining a progressive power lens according to the eighth aspect, wherein in the determination step, necessity for expanding the clear vision area is determined based on an amount of third-order aberration in the horizontal direction of the subject's eye.

According to a tenth aspect of the present invention, there is provided the method for determining a progressive power lens according to the eighth aspect, wherein a width of a smoothing filter for smoothing the power distribution in the horizontal direction is larger than a narrowest width among widths between peaks of third-order aberration on both left and right sides of a principal meridian in the progressive power lens before smoothing.

According to an eleventh aspect of the present invention, there is provided the method for determining a progressive power lens according to the eighth aspect, wherein in the design selection step, a design of a progressive power lens in which the clear vision area is expanded in the horizontal direction in exchange for increasing the amount of astigmatism on the principal meridian of the progressive power lens, is selected.

According to a twelfth aspect of the present invention, there is provided the method for determining a progressive power lens according to the eighth aspect, wherein in the design selection step, when it is determined that the subject requires expansion of the clear vision area in the horizontal direction, a design of a progressive power lens in which the clear vision area is expanded in the horizontal direction without changing an addition curve of the principal meridian, is selected.

According to a thirteenth aspect of the present invention, there is provided the method for determining a progressive power lens according to any one of the first to the twelfth aspects, wherein in the design selection step, when it is determined that the subject does not require expansion of the clear vision area, a basic design of the progressive power lens is selected in the design selection step.

### Advantage of the invention

According to one embodiment of the present invention, a progressive power lens can be determined that can be worn more comfortably by a subject who requires expansion of a clear vision area in a depth direction.

### Brief description of the drawings

FIG. 1A is a view illustrating an example of a clear vision area when a subject wearing a progressive power lens performs near vision, and is a view illustrating an example of the clear vision area in a state where there is no ocular aberration.
FIG. 1B is a view illustrating an example of the clear vision area when the subject wearing the progressive power lens performs near vision, and is a view illustrating an example of the clear vision area when an eye has a trefoil (Z[-3, 3]) in the horizontal direction.
FIG. 2 is a view illustrating an example of the clear vision area when a subject wearing a progressive power lens (addition power +2D) with a basic design and a depth expansion design according to a first embodiment of the present invention views an object on a principal meridian.
FIG. 3 is a flowchart showing an example of a method for determining the progressive power lens according to the first embodiment of the present invention.
FIG. 4 is a schematic view illustrating a line of sight movement task according to the first embodiment of the present invention.
FIG. 5 is a view illustrating an example of an original image and multiple blurred images according to the first embodiment of the present invention.
FIG. 6 illustrates an example of a spot image created by the aberration added to the blurred image according to the first embodiment of the present invention.
FIG. 7 is a view illustrating an example of addition curves in progressive power lenses with a basic design and a depth expansion design (addition power +2D) according to the first embodiment of the present invention.
FIG. 8A is a view illustrating horizontal power distributions in a near portions of the progressive power lenses (for a left eye) with a basic design and a horizontal expansion design according to the first embodiment of the present invention.
FIG. 8B is a gradient distribution view of FIG. 8A.
FIG. 9A is a view illustrating horizontal power distributions in the near portions of the progressive power lenses (for the left eye) with the basic design and a depth expansion design according to the first embodiment of the present invention.
FIG. 9B is a gradient distribution view of FIG. 9A.
FIG. 10 is a view illustrating the power distribution (Power), astigmatism distribution (As), and third-order aberration distribution (HOA, Higher Order Aberration) in the horizontal direction of the basic design of the progressive power lens according to an example of the present invention.
FIG. 11 is a view illustrating the power distribution (Power), astigmatism distribution (As), and third-order aberration distribution (HOA, Higher Order Aberration) in the horizontal direction of the progressive power lens designed to have its power distribution smoothed only in the horizontal direction, according to an example of the present invention.
FIG. 12 is a view illustrating the power distribution (Power), astigmatism distribution (As), and third-order aberration distribution (HOA, Higher Order Aberration) in the horizontal direction of a design in which the addition power of an intermediate portion of a progressive zone of the progressive power lens is reduced, according to an example of the present invention.
FIG. 13A is a view illustrating a clear vision area when a subject wearing the progressive power lens with a basic design according to an example of the present invention performs near vision, in which the left side shows a state in which the eye has no aberration, and the right side shows a state in which the eye has a 0.1 µm trefoil in a rightward direction.
FIG. 13B is a view illustrating the clear vision area when the subject wearing the progressive power lens with a horizontal expansion design according to an example of the present invention performs near vision, in which the left side shows a state in which the eye has no aberration, and the right side shows a state in which the eye has a trefoil of 0.1 µm in the rightward direction.
FIG. 13C is a view illustrating the clear vision area when the subject wearing the progressive power lens with an expanded depth design according to an example of the present invention performs near vision, in which the left side shows a state in which the eye has no aberration, and the right side shows a state in which the eye has a 0.1 µm trefoil in the rightward direction.
FIG. 14A is a view illustrating an example of a bimodal spot (left side of the figure) and a blurred image (right side of the figure) obtained by convolving the spot.
FIG. 14B is a view illustrating an example of a unimodal spot (left in the figure) and a blurred image (right in the figure) obtained by convolving the spot.

### Detailed description of the invention

### <Finding obtained by the inventors>

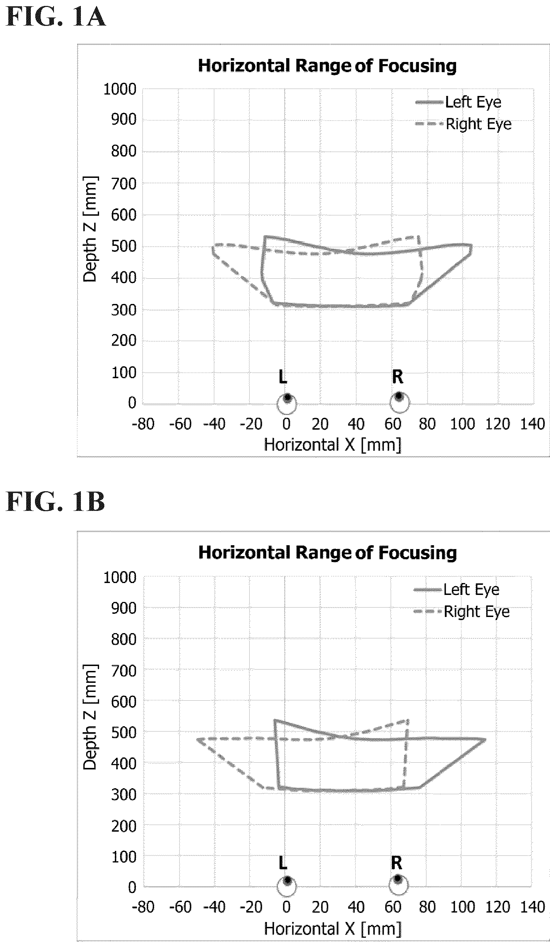

First, finding of the inventors will be described. FIG. 1A and FIG. 1B are views illustrating an example of an area where at least the direction of a Landolt ring image for a visual acuity of 0.7 is clear, or an area where the edge of a Landolt ring image for a visual acuity of 0.3 is clearly visible (hereinafter referred to as a clear vision area), when the subject wearing the progressive power lens performs near vision. In FIGS. 1A and 1B, the vertical axis indicates a front-back direction and the horizontal axis indicates a horizontal direction with respect to the left eye L of the subject. Further, the area surrounded by a dashed line is the clear vision area of the left eye L, and the area surrounded by a solid line is the clear vision area of the right eye R, and the area where both areas overlap is the clear vision area of both eyes. Hereinafter, in this specification, unless otherwise specified, the term "clear vision area" means the clear vision area of both eyes. FIG. 1A is a view illustrating an example of the clear vision area when the eye has no aberration, and FIG. 1B is a view illustrating an example of the clear vision area when the eye has a trefoil (Z[-3, 3]) third-order aberration in the horizontal direction. As illustrated in FIG. 1B, when the eye has a trefoil, it was found that the left and right clear vision areas are shifted, and the clear vision areas of both eyes become narrower. Since there is a variation in ocular aberrations depending on individuals and conditions, it is probable that the subject may not be able to wear the progressive power lens comfortably when the clear vision area is narrowed.

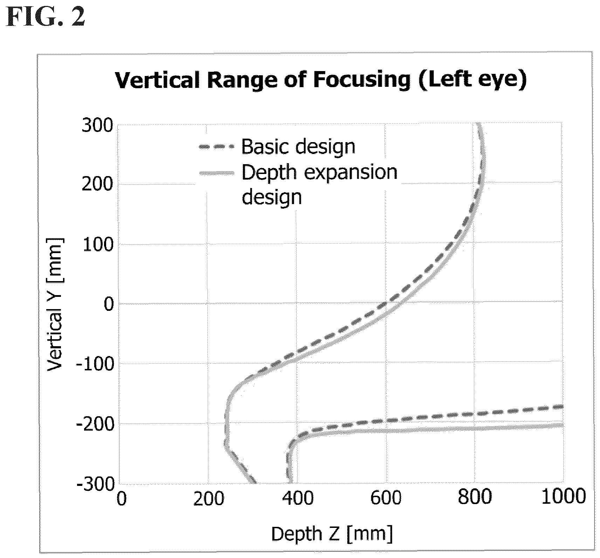

Further, FIG. 2 is a view illustrating an example of the clear vision area when the subject wearing the progressive power lens (addition power +2D) views an object on the principal meridian. In FIG. 2, the subject's left eye is set as an origin (0,0), the vertical direction is the Y axis, and the depth direction is the Z axis. The area enclosed by a dashed line in FIG. 2 indicates the clear vision area when the progressive power lens with the basic design is worn. In the optical design of the progressive power lens, the upper, middle and lower parts of a polar coordinate system with the center of rotation of the eyeball as an origin are assigned to the far, intermediate and near portions, respectively, and an object distance is also defined using the polar coordinate system. On the other hand, in terms of human spatial perception, height and depth are often sensed in a Cartesian coordinate system based on the front of a face. In terms of skelton, up and down rotation around shoulders and a depth movement by flexing and straightening arms are movements based on the polar coordinate system, but switching between facing forward and half-body position is a movement in the depth direction in the Cartesian coordinate system, resulting in a complex movement that combines both. For the Subject who is accustomed to or have adapted to a progressive multifocal lens, it is likely that his/her perception is based on the polar coordinate system with the classical center of eye rotation as an origin, and his/her rotational movement is based on fixed shoulders. On the other hand, for the subject who is not accustomed to the progressive multifocal lens or has poor adaptability, it is likely that his/her perception is based on the Cartesian coordinate system, resulting in his/her back-forth shoulder movement.

The former range of movement corresponds to an arm length, or the length from the shoulder to a little finger, and is known to be about 740 mm on average. The latter range of movement corresponds to the length of a middle finger of the arm extended horizontally from a spine, called half arm span, and is known to be about 850 mm on average. Although "arm range" is a general term, depending on the subject, it may refer to a strict definition of arm length, or it may take into account the range of back-forth shoulder movement back and forth and the range of finger extension. Therefore, it is preferable to determine in which coordinate system the subject perceives and acts, and to make design and selection accordingly. Further, many subjects who are not accustomed to the progressive multifocal lens or who have poor adaptability were found to be that although they are quick in understanding the characteristics of and adapting to areas where they can see all distances farther than a given distance, they are significantly slow in understanding the characteristics and adapting to the mid-to-near area where the range of vision is limited both to the near and far distances.

Specifically, for example, when the subject grasps and views an object at a shoulder height with his/her hands, if the object is too close or too far away and falls outside the clear vision area, for example, before and after grasping the object at a height of Y=-210 mm, which corresponds to the subject's shoulder height, the subject may not be able to wear the progressive power lens comfortably. In fact, the subjects' complaints were mostly about the difficulty in seeing when they were working at a close range with a stable posture and within a natural range of movement of the arms while sitting, but when they were picking up something a little distance away and looking at it, which involved a range of distances from medium to close, resulting in an unstable posture (for example, in real life, as soon as you pick up a newspaper, it becomes difficult to read).

The present inventors have conducted extensive research into the above-described problem. The result showed that while some subjects were not bothered by the narrowing of the clear vision area, other subjects were unable to wear the progressive power lens comfortably even when the clear vision area was not narrowed. Therefore, an index of how wide the clear vision area a subject requires (that is, whether or not the clear vision area of the basic design of the progressive power lens is sufficient, whether or not it be better to expand the clear vision area in the horizontal direction, or whether or not it be better to expand the clear vision area in the depth direction) is determined as the necessity for expanding the clear vision area, and for example, for a subject judged to require expansion of the clear vision area in the depth direction, a method is provided to select a design of the progressive power lens in which the clear vision area is expanded in the depth direction by reducing the addition power in the intermediate portion of the progressive zone of the progressive power lens, and for a subject judged to require expansion of the clear vision area in the horizontal direction, a method is provided to select a progressive power lens design in which the clear vision area is expanded in the horizontal direction by smoothing the power distribution of the progressive power lens in the horizontal direction, thereby reducing the third-order aberration in the horizontal direction around the principal meridian of the progressive power lens. There are a number of methods (parameters) for determining the necessity for expanding the clear vision area, and these will be described in detail below. This method enables to determine a progressive power lens that can be worn more comfortably with minimal risk (that is, while maintaining a basic design of the progressive power lens as much as possible) for the subject who requires expansion of the clear vision area in the depth direction or the horizontal direction.

In this specification, the basic design of the progressive power lens means the basic design of the progressive power lens such as a lens design temporarily selected (or worn) by the subject, or a lens design temporarily recommended by a seller, etc.

Regarding the third-order aberration of the progressive power lens (including trefoil and coma), it was found that in the vicinity of the near portion, the third-order aberration occurs in a manner that surrounds the principal meridian (in a direction toward the principal meridian), and that when the absolute value of the third-order aberration in the horizontal direction in the vicinity of the principal meridian becomes 0.1 µm or more larger than a position where it is at a minimum, the subject feels that near vision is difficult (for example, the direction of the Landolt ring image for a visual acuity of 0.7 is not clear, or the edge of the Landolt ring image for a visual acuity of 0.3 is not clearly visible). Accordingly, in this specification, the clear vision area may be rephrased as an area that is 0.1 µm or more larger than the position where the absolute value of the third-order aberration in the horizontal direction in the vicinity of the principal meridian is minimum.

Further, in this specification, the "front-back direction" refers to the direction of radius R in the polar coordinate system, and the "depth direction" refers to the Z direction in an XYZ orthogonal coordinate system (X is the horizontal direction, and Y is the vertical direction).

### [Details of the embodiment of the present invention]

Next, an embodiment of the present invention will be described below with reference to the drawings. The present invention is not limited to these examples, but is defined by the claims, and is intended to include all modifications within the meaning and scope of the claims.

### <First embodiment of the present invention>

### (1) Method for determining a spectacle lens

First, a method for determining a progressive power lens of this embodiment will be described. FIG. 2 is a flowchart showing an example of the method for determining a progressive power lens according to this embodiment. As shown in FIG. 2, the method for determining a progressive power lens of this embodiment includes, for example, the clear vision area necessity determination step S101 and a design selection step S102. This embodiment shows a case where the progressive power lens most suitable for a subject is determined by selecting any of the following designs: a design in which the clear vision area is expanded in the depth direction (depth expansion design); a design in which the clear vision area is expanded in the horizontal direction (horizontal expansion design) or a basic design.

### (The clear vision area necessity determination step S101)

The clear vision area necessity determination step S101 is a step of determining the necessity for expanding the clear vision area for a subject wearing the progressive power lens (to which the basic design is applied). There are a number of methods (parameters) for determining the necessity for expanding the clear vision area. An example of such a method will be described below.

In the clear vision area necessity determination step S101, the necessity for expanding the clear vision area may be determined based on, for example, the amount of third-order aberration in the horizontal direction of the subject's eye. As shown in FIG. 1B, when the eye has third order aberration (trefoil), the clear vision area is narrowed. Accordingly, it may be determined that the subject whose third-order aberration of eyes in the horizontal direction (absolute value of a difference between left and right) is large (for example, 0.1 µm or more) requires expansion of the clear vision area in the horizontal direction. Further, when the subject suffers from dry eye or the like, the trefoil in the eye is likely to increase, so the measured value of the third-order aberration may be adjusted, for example, taking into account a subject's constitution. The amount of third-order aberration of the subject's eye can be measured using a known technique such as a wavefront sensor.

However, since there is a great variation in the amount of third-order aberration of the eye depending on the condition of the subject and the surrounding environment, it is preferable to determine the necessity for expanding the clear vision area taking other parameters into consideration. Other parameters for determining the necessity for expanding the clear vision area will be described below.

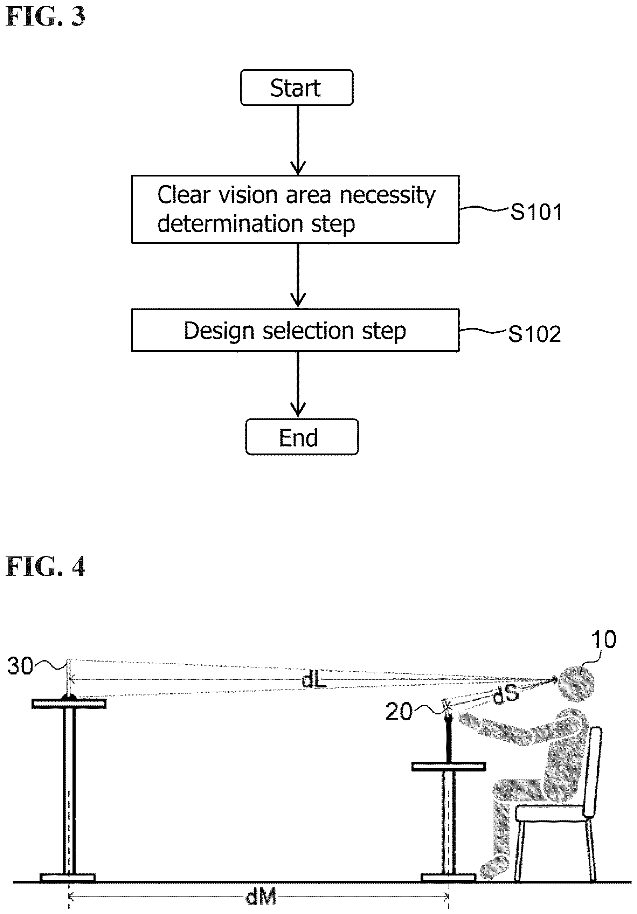

In the clear vision area necessity determination step S101, for example, the necessity for expanding the clear vision area may be determined based on the transition of the subject's line of sight movement when the subject alternates between near vision and far vision. FIG. 4 is a schematic view for explaining a task for determining the necessity for expanding the clear vision area based on the transition of the subject's line of sight movement (hereinafter referred to as a line of sight movement task). As illustrated in FIG. 4, in the line of sight movement task, a subject 10 repeatedly (e.g., alternately) views a near target 20 placed near (e.g., visual distance dS = 40 cm) and a far target 30 placed far (e.g., visual distance dL = 3 m). As the near target 20 and the far target 30, for example, a Landolt ring image for visual acuity of 0.7 displayed on a display terminal such as a tablet can be used.

When the subject can clearly view the near target and the subject's line of sight movement is small (e.g., equivalent to reading in real life), the size of the clear vision area does not matter much. However, for example, when the subject moves the line of sight from the far target to the near target (e.g., when the subject moves the line of sight from a TV screen to a book in his/her hand in real life), some subjects find it difficult to focus on the near target when the clear vision area is narrow. Specifically, this includes the subjects who are not accustomed to using the progressive power lens and use different various points on the lens when viewing the near target (i.e. subjects having large variations in head movement and convergence), or subjects who take a long time (response time) to clearly view the near target. Accordingly, the line of sight movement task may be performed, and it may be determined that the subject who performs the line of sight movement task and has a large variation in head movement and convergence (especially when moving from far to near vision) requires expansion of the clear vision area in the horizontal direction. Further, when the subject performs the line of sight movement task and has a long (or large variation in) response time until he/she can clearly view the near target when moving from far to near vision for example, it may be determined that this subject requires expansion of the clear vision area in the horizontal direction (or in the depth direction). Further, when the subject performs a line of sight movement task, and for example, has a large difference in response time between moving from far vision to near vision and from near vision to far vision, it may be determined that this subject requires expansion of the clear vision area in the depth direction. When measuring the amount of head movement and convergence of the subject in the line of sight movement task, for example, the subject's head and eyeballs are imaged using a camera attached to a display device that displays the near target, and the amount of head movement and convergence can be measured by image recognition.

Further, in the clear vision area necessity determination step S101, for example, the necessity for expanding the clear vision area may be determined based on the subject's sensitivity to aberration. In the design selection step S102 described later, when the progressive power lens design in which the clear vision area is expanded in the horizontal direction or depth direction is selected, the amount of astigmatism on the principal meridian increases compared to the basic design. That is, in exchange for expanding the clear vision area in the horizontal direction or depth direction, the quality (image quality) on the principal meridian will be reduced compared to a basic design. Accordingly, for the subject who is sensitive to an increase in the amount of astigmatism on the principal meridian, the progressive power lens with a basic design, without expanding the clear vision area in the horizontal direction or depth direction, may be more comfortable to wear. On the other hand, for the subject who cannot sense the increase in the amount of astigmatism on the principal meridian, the progressive power lens with a horizontal expansion design or depth expansion design may be more comfortable to wear.

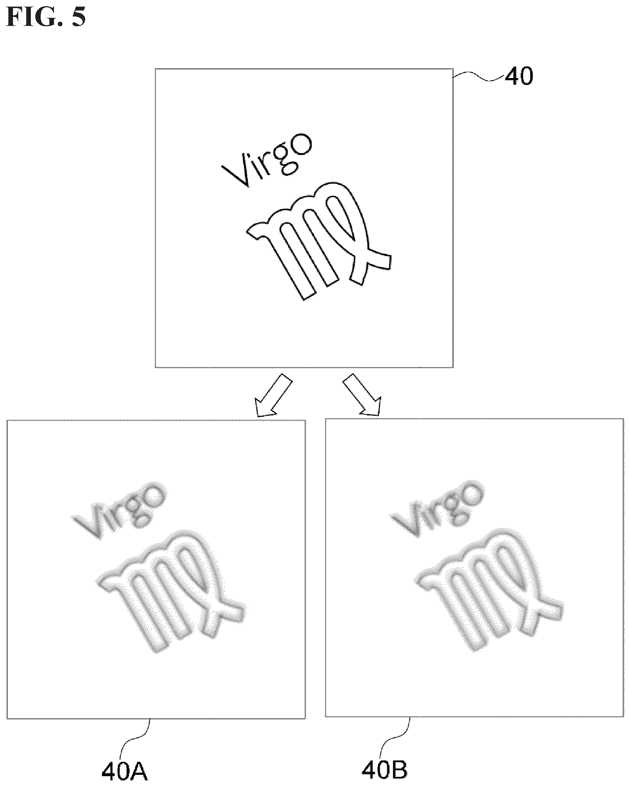

An example of a method for measuring a subject's sensitivity to aberration will be described. To measure the sensitivity to aberration, for example, multiple blurred images (in this embodiment, two blurred images) in which the amount of added aberration (preferably only that amount) is varied relative to a predetermined original image, can be used. FIG. 5 illustrates an example of the original image and multiple blurred images. FIG. 5 illustrates a blurred image 40A obtained by adding a predetermined amount of aberration to an original image 40, and a blurred image 40B obtained by adding a larger amount of aberration than the blurred image 40A.

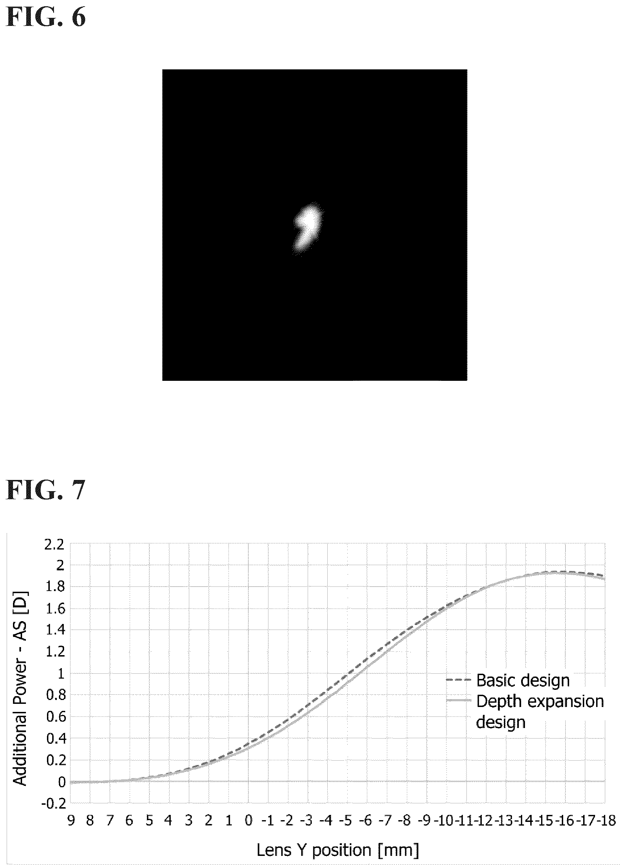

FIG. 6 illustrates an example of a spot image created by the aberration added to the blurred image, and illustrates the spot image with a trailing tail at the bottom left of the page. The blurred image to which aberration that creates such a spot image has been added appears blurred in the lower left direction. In other words, the spatial frequency characteristics in the lower left direction are significantly (characteristically) decreased due to the added aberration. Such a characteristic direction of the decrease of the spatial frequency characteristics due to the added aberration is referred to as a direction of aberration in this specification.

It is preferable that the directions of the aberrations added to multiple blurred images (for example, the blurred image 40A and the blurred image 40B) are substantially the same. This makes it easier for the subject to compare the appearance of the multiple blurred images. In this specification, the term "the directions of aberration are substantially the same" includes a case where the directions are completely the same, as well as a case where there is a slight difference of ±15 degrees or less in the direction of aberration. The direction of aberration refers to the direction of the component with a largest absolute value among all the coefficients of all the blurred images when the aberrations added to the blurred images displayed simultaneously are expanded into Zernike polynomials.

When selecting the aberration to be added to the original image 40, for example, it may be arbitrarily selected from among the aberrations caused by a standard progressive power lens.

After multiple blurred images are prepared, the subject is presented with the multiple blurred images simultaneously and asked to compare the appearances of them, and the subject's subjective response is obtained to measure the subject's sensitivity to aberration. Specifically, for example, a task (hereinafter referred to as an aberration sensitivity task) is performed in which blurred images 40A and 40B as illustrated in FIG. 5 are simultaneously presented to the subject to compare the appearances of them, and the subject is asked to select which one appears clearer (or which one is "unclear"). Then, when the subject selects the blurred image 40A with a small amount of added aberration (i.e., the correct answer), it may be determined that the subject has high sensitivity to blur. When the subject selects the blurred image 40B with a large amount of added aberration (i.e., the incorrect answer), it may be determined that the subject has low sensitivity to blur. In this specification, presenting multiple blurred images simultaneously means presenting multiple blurred images so that the multiple blurred images are visible within the field of view of the subject, and there is no limitation in the timing of the start or end of presenting each blurred image (for example, there may be a deviation in the timing of the start or end of presenting the blurred image 40A and the blurred image 40B).

However, when the sensitivity to blur is determined based on a single subjective response, for example, this may include a case where the blurred image with a small amount of aberration happens to be selected even when the difference in appearance is not actually known. Therefore, in this embodiment, it is preferable to further prepare multiple similar blurred images similar to the plurality of blurred images, then, simultaneously present the multiple similar blurred images to a subject, in which the amount of added aberration (preferably only that amount) is varied, then, allow the subject to compare the appearances of them, and obtain the subject's subjective response multiple times to measure the stability of the subjective response. The similar blurred image means, for example, an image obtained by rotating a blurred image at an arbitrary angle, or an image obtained by enlarging or reducing the blurred image at an arbitrary magnification.

When the subject is subjected to the above-described aberration sensitivity task and determined to have a high sensitivity to blur, it may be determined that the subject does not require expansion of the clear vision area in the horizontal direction or depth direction (or that the disadvantage of selecting the horizontal expansion design or depth expansion design is likely to outweigh the advantage). Further, for the subject who is determined to have a low sensitivity to blur, it may be determined that the subject requires expansion of the clear vision area in the horizontal direction or depth direction (or that the advantage of selecting the horizontal expansion design or depth expansion design is likely to outweigh the disadvantage).

Further, the aberration sensitivity task may be performed multiple times, and for the subject who stably gives correct answers, it may be determined that the subject has a high sensitivity to blur, and a low aberration design may be selected in the design selection step S102. Further, for the subject who stably gives incorrect answers, it may be determined that the subject has a high sensitivity to blur but prefers a state with large aberration, and a design having the aberration preferred by the subject may be selected in the design selection step S102. Further, for the subject who gives unstable answers (correct or incorrect is not stable) and who answers "I don't know" only a few times, it is determined that the subject has a low sensitivity to blur but does not consciously recognize that he or she has a low sensitivity, and in the design selection step S102, a balanced design between a low aberration design and a design with other improvement (for example, a design that expands the clear vision area in the horizontal directions) may be selected. Further, for the subject who frequently answers "I don't know," it may be determined that they have a low sensitivity to blur, and an improved design that trades off aberration (for example, a design that expands the clear vision area in the horizontal direction) may be selected.

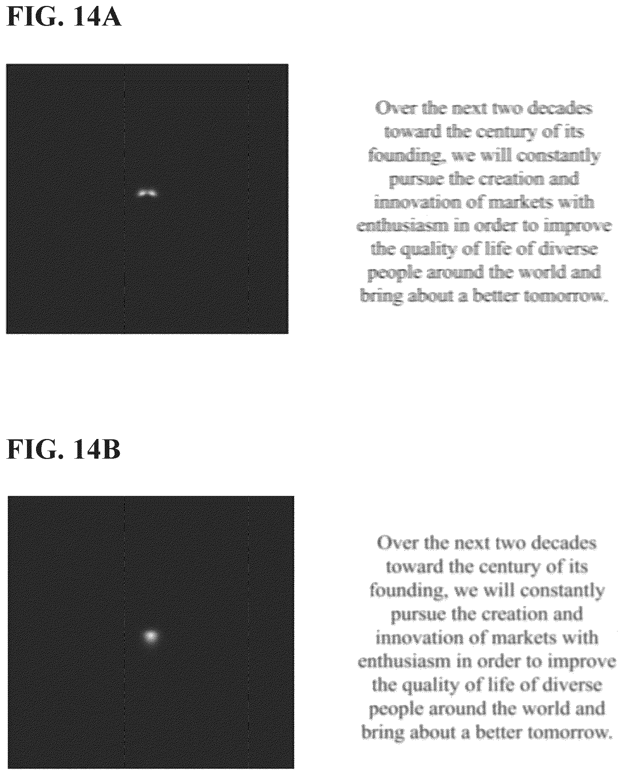

Further, as a result of further investigation by the inventors, it was found that the subject does not judge the visibility of an image solely based on the simple size of blur (or the amount of aberration that causes blur) or MTF (Modulation Transfer Function). A specific example is illustrated in FIG. 14A and FIG. 14B. In FIG. 14A and FIG. 14B, the blurred image in which blur corresponding to the spot shown on the left is added (also called convoluting the spot) is shown on the right. The spot illustrated in FIG. 14A has a smaller spread (that is, a smaller sum of squares of the aberration amount) than the spot illustrated in FIG. 14B, but has a bimodal characteristic. On the other hand, the spot illustrated in FIG. 14B has a single peak, although the spot spreads somewhat widely. A group of people were asked to compare the appearances of the blurred images illustrated in FIGS. 14A and 14B. Then, it was found that since the blurred image in FIG. 14A has clear lines but is doubled, most of the subjects answered that the blurred image in FIG. 14B was relatively more preferable. This is considered to be because very few subjects preferred bimodal blur. Accordingly, in the aberration sensitivity task, the subject may be asked to compare a blurred image having a slightly small amount of aberration but convolved with a multi-modal (including bimodal equivalent to astigmatism plus spherical aberration of the eye, trimodal equivalent to trefoil plus spherical aberration of the eye, and quadrupal equivalent to higher-order aberrations of the eye) spot, with a blurred image having a slightly large amount of aberration but convolved with a unimodal spot, and a subject who prefers the blurred image having a unimodal spot may be determined to have a high sensitivity to blur. Here, "high sensitivity to blur" refers to sensitivity to whether or not aberration correction is performed correctly, rather than sensitivity to the amount of aberration.

In the aberration sensitivity task, the subject's subjective response may be scored depending on whether it coincides with an average support in the group of people, and the subject's sensitivity to blur may be determined based on the score. The average support may be calculated using a statistical value obtained when a question is given to a certain group in advance, or may be estimated using machine learning, etc. Specifically, for example, in a question that asks the subjects to compare left and right blurred images, if many subjects prefer the left blurred image (the support rate is high), the subjects who answered that the left image is preferred may be given +1 point or the like since it coincides with the average support in the group, and the subjects who answered that the right image is preferred may be given -1 point or the like since it does not coincide with the average support in the group. Then, the total score and subtotals for each problem tendency may be tallied, and for example, when the score is above a certain score (threshold value), it may be determined that the sensitivity to blur is high, and when the score is less than the certain score (threshold value), it may be determined that the sensitivity to blur is low. That is, the subject's sensitivity to blur may be determined based on a large and small relationship between the score and a predetermined threshold value. By assigning scores in this manner, it is possible to make a judgment not only based on the simple size or amount of aberration or MTF, but also taking into consideration the subject's preference for blur (the tendency and degree of blur that is acceptable to the subject). Further, the characteristics of the subject become easier to understand, making it easier to provide counseling, etc., when determining on a spectacle lens.

Further, in the clear vision area necessity determination step S101, for example, the necessity for expanding the clear vision area may be determined by performing a task (hereinafter referred to as an object movement task) that includes the actions of holding, viewing, and moving an object (e.g., a smartphone) equipped with a gyro sensor (angular velocity sensor) and capable of acquiring position information, and based on the movement transition of the object during the object movement task. Specifically, in a state where the subject can clearly view the object, the subject is asked to move the object while keeping a visual distance constant, or to move the object while changing the visual distance, and for the subject who can move the object smoothly, it may be determined that the subject does not require expansion of the clear vision area in the depth direction. On the other hand, for the subject who is unable to move the object smoothly, it may be determined that the subject requires expansion of the clear vision area in the depth direction.

Further, analysis may be performed whether the movement of the object is due to rotation or radial movement around arms or eyes, or due to vertical and horizontal movement in the Cartesian coordinate system, including back-forth movement of the shoulders and face, and in the former case, it may be determined that the subject does not require expansion of the clear vision area in the depth direction, and in the latter case, it may be determined that expansion of the clear vision area is required.

Further, in determining the smoothness of the object movement, it may be determined based on the time from when the subject starts the object movement task without holding the object in his/her hand until the subject grasps the object and first see it, and line of sight, swaying of the head and shoulders, etc. Particularly, for the subject whose posture is changed significantly before and after reaching out toward the object and then returning, the position of the line of sight movement on the lens is prone to fluctuation together with the significant change of posture, and therefore it is preferable to determine that the subject has poor depth perception and requires expansion of the clear vision area in the depth direction.

The movement transition may be measured using a sensor other than the gyro sensor. For example, an object with an embedded IC chip may be moved to measure the movement transition from outside using electromagnetic waves. Alternatively, an object having no measurement mechanism may be used to perform measurements from outside using optical distance measurement.

Additionally, in the clear vision area necessity determination step S101, the necessity for expanding the clear vision area may be determined taking into account a subject's lifestyle, such as a subject's occupation, hobbies, etc. For example, for the subject whose occupation often requires them to perform a certain movement within an arm's length or a short distance away while maintaining a fixed posture, it may be determined that the subject requires expansion of the clear vision area in the depth direction.

Further, the necessity for expanding the clear vision area may be determined by combining the various parameters described above. For example, in the clear vision area necessity determination step S101, the necessity for expanding the clear vision area may be determined based on at least one (preferably two, more preferably three, further preferably four) of the amount of third-order aberration in the horizontal direction of the subject's eye, the transition of the subject's line of sight movement when the subject is asked to repeatedly switch between near and far vision, the subject's sensitivity to aberration, and the movement transition of the object during the object movement task.

Of the methods (parameters) for determining the necessity for expanding the clear vision area described above, the method based on the amount of horizontal third-order aberration of the subject's eye, the method based on the transition of the subject's line of sight movement when the subject is asked to repeatedly perform near vision and far vision, and the method based on the subject's sensitivity to aberration are suitable for determining whether or not the subject requires expansion of the clear vision area in the horizontal direction. Also, the method based on the transition of the subject's line of sight movement when the subject repeatedly switches between near and far vision, and the method based on the transition of the object's movement when the object movement task is performed, are suitable for determining whether or not the subject requires expansion of the clear vision area in the depth direction Accordingly, in the clear vision area necessity determination step S101, for example, based on the result of determination using one method or the result of a prior interview with the subject, in which direction, horizontal or depth, the subject is most likely to require expansion of the clear vision area, and then whether or not to adopt another method may be determined.

### (Design selection step S102)

In the design selection step S102, for example, in the clear vision area necessity determination step S101, when it is determined that the subject requires expansion of the clear vision area in the depth direction, this is the step for selecting a design of the progressive power lens in which the clear vision area is expanded in the depth direction by reducing the addition power in the intermediate portion of the progressive zone of the progressive power lens. This step enables to determine the progressive power lens that can be worn more comfortably by the subject who requires expansion of the clear vision area in the depth direction. In the design selection step S102, for example, a lens having a design suitable for the subject may be selected from a plurality of customized lenses prepared in advance.

The design of the progressive power lens in which the clear vision area is expanded in the depth direction (depth expansion design) will be described more specifically. A subject with an average build has his/her shoulders 210 mm below an eye position, and when he/she pushes his/her shoulders out and stretches his/her arms horizontally, his/her middle fingertip reaches 850 mm away, so this position is preferably within the clear vision area. This assumes that when the subject of average build picks up an object, the moment they see and touch the object occurs within the clear vision area. That is, in the depth expansion design, it is preferable that the position of Y=-210 mm, Z=850 mm is within the clear vision area, as shown by the area surrounded by the solid line in FIG. 2. In this state, when the clear vision area is defined in the Cartesian coordinate system that is along a body axis or in the polar coordinate system based on a shoulder, the clear vision area is expanded to a full arm's length in the depth or front-back direction of the coordinate system, and therefore the subject is able to more easily perceive his/her clear vision area and is more comfortable wearing the progressive power lens. In this specification, expanding the clear vision area in the depth direction means expanding the area in a vertical direction (especially downward) that can be seen up to the arm's stretched length (Z=850 mm) or to infinity. This does not mean that the physical quantity of the lens design is expanded, but rather that the length along a depth vector (either eye-based or horizontal-based) for the subject is expanded.

Further, when the subject of average build faces forward and stretches his/her arm horizontally, the tip of the little finger is located 740 mm away, and it is preferable that this position is also within the clear vision area. This assumes that when the subject of average build picks up an object, he/she will be able to grasp and hold it firmly within the clear vision area.

The conditions for setting the position of Y=-210 mm, Z=850 mm within the clear vision area will be described. When the distance from the lens to the center of an eyeball rotation is 27 mm, then the position on the lens corresponding to a visual angle tan θ=-210/850 is 27 mm×tan θ=-6.67 mm, which is a position -6.67 mm from a fitting point. Accordingly, at this position, the addition power taking astigmatism into account (addition power - absolute value of astigmatism/2) may be less than 1000/√(210² + 850²) = 1.14 D, which is a diopter conversion value for a distance to the position of Y = -210 mm, Z = 850 mm.

FIG. 7 is a view illustrating an example of addition curves of the progressive power lenses (addition power +2D) with a basic design and a depth expansion design. In FIG. 7, the horizontal axis indicates the vertical position with a lens fitting point being 0, and the vertical axis indicates the addition power taking astigmatism into account (addition power-absolute value of astigmatism/2). As illustrated in FIG. 7, in the depth expansion design, the addition power in the intermediate portion of the progressive zone (eg, around Y=-6.67 mm) is reduced compared to the basic design. Thereby, the addition power taking into account astigmatism at position Y = -6.67 mm (addition power - absolute value of astigmatism/2) can be less than 1.14 D, and as illustrated in FIG. 2, position Y = -210 mm, Z = 850 mm can be set within a clear vision area. In the case of a progressive power lens with an addition power of less than +2D, it is preferable to reduce the addition power in the intermediate portion of the progressive zone so that the addition power taking into account astigmatism at position Y=-6.67 mm (addition power-absolute value of astigmatism/2) is 1.14 x (addition power/2). Thereby, for example, a boundary on the lower end side of the clear vision area illustrated in FIG. 2 approaches a right-sloping slope, and therefore the range that can be seen to infinity can be wider.

As described above, by reducing the addition power in the intermediate portion of the progressive zone of the progressive power lens, there is an advantage in that the clear vision area is expanded in the depth direction, but there is also a disadvantage in that the amount of astigmatism on the principal meridian increases compared to the basic design. Accordingly, in the design selection step S102, the design of the progressive power lens can be selected in which the clear vision area is expanded in the depth direction in exchange for increasing the amount of astigmatism on the principal meridian of the progressive power lens.

Further, as described above, by reducing the addition power in the intermediate portion of the progressive zone of the progressive power lens, the third-order aberration in the horizontal direction around the principal meridian of the progressive power lens can be slightly reduced, so that the clear vision area is expanded not only in the depth direction but also in the horizontal direction. Accordingly, in the design selection step S102, by reducing the addition power in the intermediate portion of the progressive zone of the progressive power lens, the design of the progressive power lens can be selected in which the clear vision area is expanded in both the depth direction and the horizontal directions. When it is determined that the subject more requires expansion of the clear vision area in the horizontal direction than in the depth direction, it is preferable to select the horizontal expansion design that has a greater effect of expanding the clear vision area in the horizontal direction.

In the design selection step S102, for example, when it is determined that the subject requires expansion of the clear vision area in the horizontal direction in the clear vision area necessity determination step S101, the design of the progressive power lens may be selected in which the power distribution of the progressive power lens is smoothed in the horizontal direction to reduce third-order aberration in the horizontal direction around the principal meridian of the progressive power lens, and the clear vision area is expanded in the horizontal direction. This allows to determine a progressive power lens that is more comfortable to wear for the subject who requires expansion of the clear vision area in the horizontal direction.

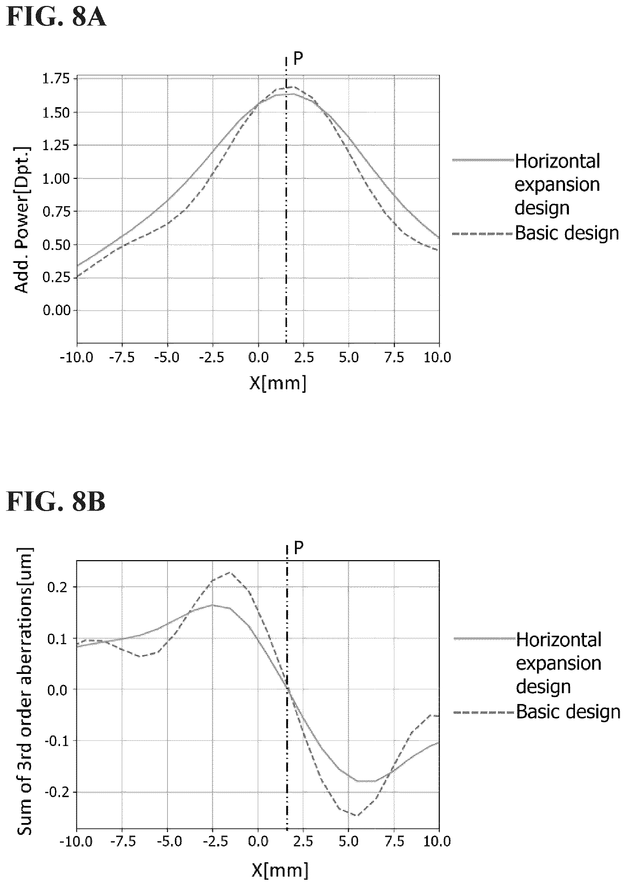

The design of the progressive power lens in which the clear vision area is expanded in the horizontal direction (horizontal expansion design) will be described more specifically. FIG. 8A is a view illustrating the power distribution in the horizontal direction of the near portion of the progressive power lens (for the left eye), and FIG. 8B is a view illustrating a gradient distribution of FIG. 8A. The total value of the coma aberration in the 0 degree direction and the trefoil is proportional to this gradient distribution. As illustrated in FIG. 8A, in contrast to the basic design (dashed line), in the horizontal expansion design (solid line), the power distribution around the principal meridian P is smoothed by applying a smoothing filter (eg, a Gaussian filter) in the horizontal direction. When the degree of the principal meridian is reduced by smoothing, the degree of the principal meridian can be adjusted to be the same (or approximately the same) as that of the basic design by offsetting the reduction in a sag direction. As illustrated in FIG. 8B, by smoothing the power distribution in the horizontal direction, the third-order aberration distribution in the horizontal direction also becomes gentler, and therefore the clear vision area (for example, the area around the principal meridian P where the amount of third-order aberration is within ±0.1 µm) is expanded in the horizontal direction. By selecting such a horizontal expansion design, a progressive power lens can be determined that can be worn more comfortably with minimal risk (i.e., while maintaining a basic design of the progressive power lens as much as possible) for a subject who requires expansion of the clear vision area in the horizontal direction.

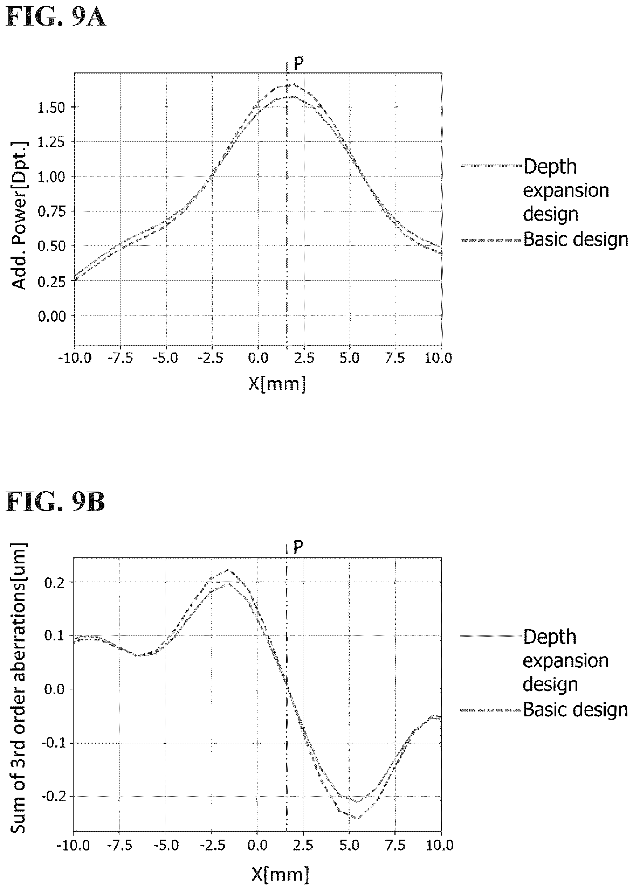

Further, as described above, it will be explained that the depth expansion design also has the effect of expanding the clear vision area in the horizontal direction. FIG. 9A is a view illustrating a horizontal power distribution in the near portion of a progressive power lens (for the left eye), and FIG. 9B is a view illustrating a gradient distribution of FIG. 9A. The sum of coma aberration and trefoil in the 0 degree direction is proportional to this gradient distribution. As illustrated in FIG. 9A, in contrast to the basic design (dashed line), the power distribution around the principal meridian P is smoothed also in the depth expansion design (solid line). When the degree of the principal meridian is reduced by smoothing, the degree of the principal meridian can be adjusted to be the same (or approximately the same) as in the basic design by offsetting the reduction in the sag direction. As illustrated in FIG. 9B , in the depth expansion design also, the power distribution is smoothed in the horizontal direction, and the third-order aberration distribution in the horizontal direction is also smoothed, and therefore the clear vision area (for example, the area around the principal meridian P where the amount of third-order aberration is within ±0.1 µm) is expanded in the horizontal direction.

It is preferable that the width of the smoothing filter for smoothing the power distribution of the progressive power lens in the horizontal direction is larger than a narrowest width among the widths between the peaks of third-order aberration on both the left and right sides of the principal meridian in the progressive power lens before smoothing. Thereby, the third-order aberration can be effectively reduced.

As described above, by smoothing the power distribution of the progressive power lens only in the horizontal direction, there is an advantage of reducing the third-order aberration in the horizontal direction around the principal meridian of the progressive power lens and expanding the clear vision area in the horizontal direction, but there is also a disadvantage of increasing the amount of astigmatism on the principal meridian compared to a basic design. Accordingly, in the design selection step S102, it is also acceptable to select the design of the progressive power lens in which the clear vision area is expanded in the horizontal direction in exchange for increasing the amount of astigmatism on the principal meridian of the progressive power lens.

When it is determined that the subject requires expansion of the clear vision area in the horizontal direction, in the design selection step S102, it is preferable to select the design of the progressive power lens in which the clear vision area is expanded in the horizontal direction by smoothing the power distribution only in the horizontal direction without changing the addition curve of the principal meridian. Thereby, vertical increase in aberration is reduced, and the clear vision area can be expanded in the horizontal direction while maintaining the basic design of the progressive power lens as much as possible.

Further, in the clear vision area necessity determination step S101, when it is determined that the subject does not require expansion of the clear vision area in the horizontal direction and depth direction (or that the disadvantage of selecting the horizontal expansion design or depth expansion design is likely to outweigh the advantage), the basic design of the progressive power lens may be selected without selecting the horizontal expansion design or depth expansion design. Thereby, the progressive power lens with optimized quality (image quality) on the principal meridian can be provided for the subject who does not require expansion of the clear vision area in the horizontal direction or depth direction.

By applying the above-described method to multiple subjects, the progressive power lens designs suited to the needs of each individual subject can be provided.

### <Other embodiments of the present invention>

The embodiment of the present invention has been specifically described above. However, the present invention is not limited to the above-described embodiment, and various modifications can be made without departing from the gist of the present invention.

For example, in the above embodiment, the method for determining a progressive power lens has been described, but the present invention can also be applied as a method for determining the necessity for expanding the clear vision area. In this case, only the clear vision area necessity determination step S101 may be performed, and the design selection step S102 may be omitted.

Further, for example, the above-described embodiment shows a case where any one of the horizontal expansion design, the depth expansion design or the basic design is selected, but the horizontal expansion design and the depth expansion design are not limited to one, and may be selected from a number of variations (e.g., design in which the width of the clear vision area differs in the horizontal direction).

Further, for example, the above-described embodiment shows a case where a design selection is performed according to the third-order aberration of the eyeball. However, the design selection may also be performed according to (or taking into account) the degree of fixation disparity or the degree of deviation between the line of sight of the eye and a visual axis. This is because the amount of the third-order aberration component in the horizontal direction is adjusted based on the degree of the deviation from the measured ocular aberration. In addition to the degree of deviation, elements of horizontal decentering from a standard model in design or measurement are factors that cause fluctuations in the amount of third-order aberration, and are therefore preferably used in determining a design selection.

### Examples

Next, examples of the present invention will be described. These examples are merely examples of the present invention, and the present invention is not limited to these examples.

### (The clear vision area necessity determination step S101)

In this embodiment, the above-described eye movement task was performed, and the necessity for expanding a clear vision area was determined based on the transition of the subject's eye movement. Specifically, as illustrated in FIG. 4, a near target 20 (a Landolt ring image for visual acuity of 0.7 displayed on a smartphone) was placed 30 degrees below the subject 10 at a visual distance dS = 40 cm, and a far target 30 (a Landolt ring image for visual acuity of 0.7 displayed on a tablet device) was placed in front of the subject 10 at a visual distance of dL = 3 m. Then, the near target 20 and the far target 30 were alternately displayed 30 times in total, and the subject 10 was asked to visually recognize and answer the gaps in the Landolt ring image.

In this example, when the subject's head movement and convergence amount varied greatly when performing the line of sight movement task, or when the response time was long (or varied greatly), it was determined that the subject required the expansion of the clear vision area in the horizontal direction. When there was a small variation in head movement and convergence, but a large difference in response time between moving from far vision to near vision and moving from near vision to far vision, it was determined that the subject required expansion of the clear vision area in the depth direction. When none of the above applies, it was determined that the subject required the expansion of the clear vision area in the horizontal and depth directions.

The above determination is based on a correlation with a level of ability to see through a clearly visible point on the lens, but some subjects may move their head back and forth due to habit, regardless of whether they are wearing the progressive power lens or not. In order to respond to such a case, a hearing may be conducted in advance and this may be taken into consideration in the determination. For the subject who has the habit of moving his/her head back and forth, the depth expansion design is often preferable to reduce the effects of distance movement and blurring of the line of sight caused by head movement. Since an optimal design is often not determined by simple head movement alone, it is preferable to make determination based on multiple pieces of information and multiple tasks.

### (Design selection step S102)

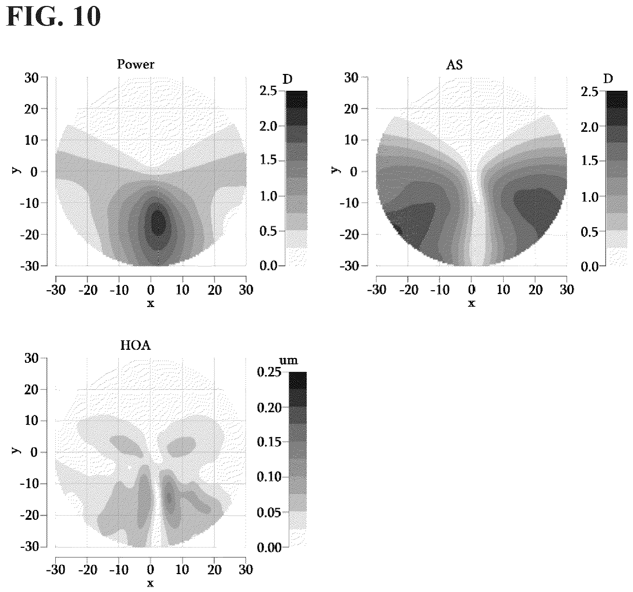

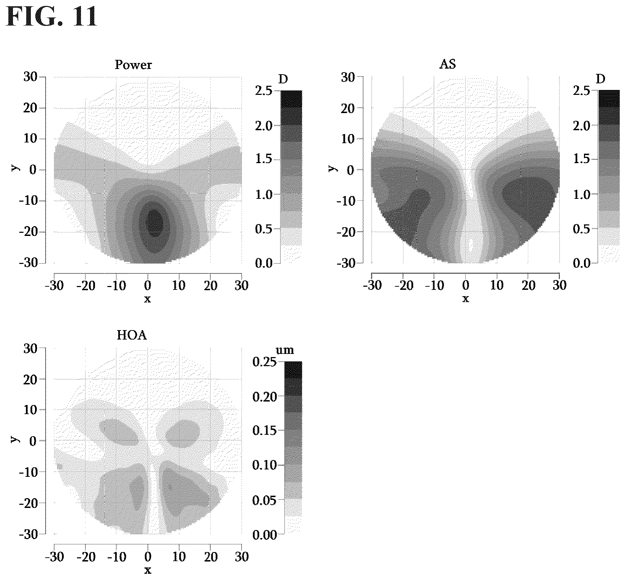

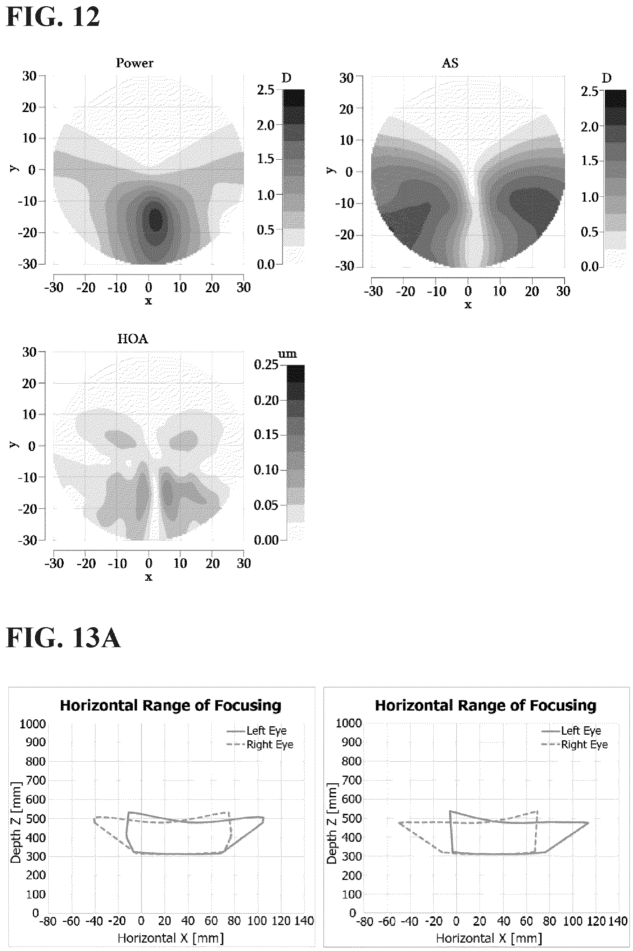

FIG. 10 is a view illustrating the power distribution (Power), astigmatism distribution (As), and third-order aberration distribution (HOA, Higher Order Aberration) in the horizontal direction of the basic design of the progressive power lens. In this example, when it was determined that the subject did not require expansion of the clear vision area in the horizontal direction and depth direction, this basic design was selected. Further, FIG. 11 is a view illustrating the power distribution (Power), astigmatism distribution (As), and third-order aberration distribution (HOA, Higher Order Aberration) in the horizontal direction of the progressive power lens designed to have its power distribution smoothed only in the horizontal direction. In this example, when it was determined that the subject required expansion of the clear vision area in the horizontal direction, this design (horizontal expansion design) was selected. Further, FIG. 12 is a view illustrating the power distribution (Power), astigmatism distribution (As), and third-order aberration distribution (HOA, Higher Order Aberration) in the horizontal direction in the design in which the addition power in the intermediate portion of the progressive zone of the progressive power lens is reduced. In this example, when it was determined that the subject required expansion of the clear vision area in the depth direction, this design (depth expansion design) was selected. The third-order aberration distributions shown in FIGS. 10 to 12 show the sum of the squares of the 0 degree component of coma and the 0 degree component of trefoil.

A illustrated in FIGS. 10 and 11, it was confirmed that in the horizontal expansion design, in exchange for slightly increasing the amount of astigmatism on the principal meridian compared to the basic design, the amount of third-order aberration in the horizontal direction around the principal meridian was reduced. Further, as illustrated in FIG. 10 and FIG. 12, it was also confirmed that in the depth expansion design, compared to the basic design, the amount of third-order aberration in the horizontal direction around the principal meridian was slightly reduced in exchange for slightly increasing the amount of astigmatism on the principal meridian.

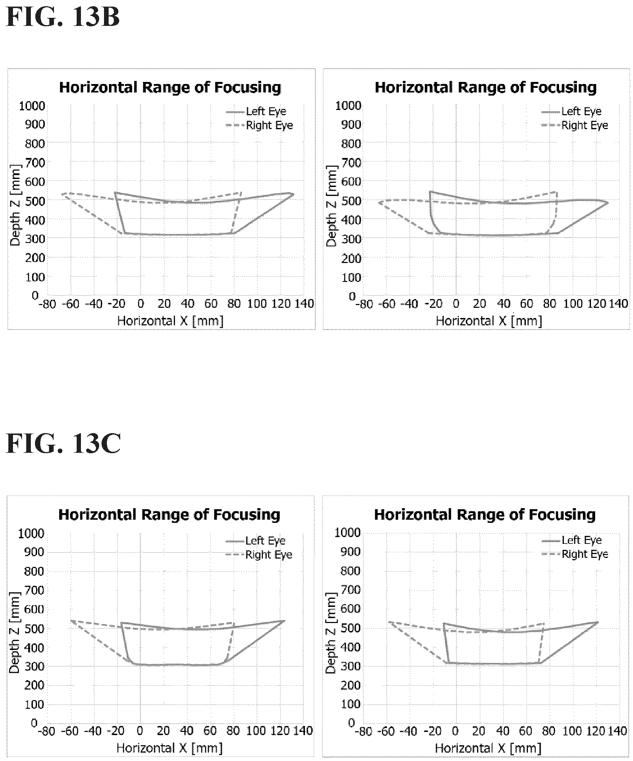

FIG. 13A is a view illustrating a clear vision area (an area where at least the direction of a Landolt ring image for visual acuity of 0.7 can be determined, or an area where the edge of the Landolt ring image for visual acuity of 0.3 is clearly visible) when the subject wearing the progressive power lens with a basic design performs near vision, in which the left side shows the eye in a state without aberration, and the right side shows the eye in a state with a 0.1 µm trefoil in the rightward direction. FIG. 13B is a view illustrating the clear vision area when the subject wearing the progressive power lens with a horizontal expansion design performs near vision, in which the left side shows a state in which there is no aberration in the eye, and the right side shows a state in which the eye has a trefoil of 0.1 µm in the rightward direction. FIG. 13C is a view illustrating the clear vision area when the subject wearing the progressive power lens with a depth expansion design performs near vision, in which the left side shows a state in which the eye has no aberration, and the right side shows a state in which the eye has a 0.1 µm trefoil in the rightward direction. In FIGS. 13A to 13C, the vertical axis indicates a front-back direction and the horizontal axis indicates a horizontal direction with respect to the subject's left eye. The area surrounded by the dashed line is the clear vision area of the left eye, and the area surrounded by the solid line is the clear vision area of the right eye, and the area where these two overlap is the clear vision area of both eyes.

As illustrated in FIGS. 13A and 13B, it was confirmed that the clear vision area was expanded in the horizontal direction in the horizontal expansion design compared to a basic design. It was also confirmed that the clear vision area was expanded in the horizontal direction when the subjects' eye had trefoil. It was also confirmed that the effect of expanding the clear vision area caused by the horizontal expansion design was more remarkable when the subjects' eye had trefoil. It was also confirmed that, as illustrated in FIG. 13A and FIG. 13C, in the depth expansion design also, the clear vision area was expanded in the horizontal direction compared to the basic design.

As explained in the first embodiment, in order for the position of the middle fingertip when the subject sticks out his/her shoulder and stretches his/her arm horizontally (position Y=-210 mm, Z=850 mm in FIG. 2) to be within the clear vision area, the addition power taking into account astigmatism (addition power-absolute value of astigmatism/2) at a position on the lens that is -6.67 mm from the fitting point may be less than 1.14 D. In the basic design of this embodiment, the addition power taking astigmatism into account was 1.211 D, which did not satisfy the above condition. On the other hand, in the depth expansion design of this embodiment, the addition power taking astigmatism into account was 1.135 D, and it was confirmed that the above condition was satisfied. That is, in the depth expansion design, it was confirmed that the position of Y=-210 mm, Z=850 mm was within the clear vision area.

As described above, it was confirmed that when the necessity for expanding the clear vision area is determined for the subject wearing the progressive power lens and it is determined that the subject requires expansion of the clear vision area in the depth direction, then, by selecting the depth expansion design, the progressive power lens that is worn more comfortably can be determined. It was also confirmed when the necessity for expanding the clear vision area is determined for the subject wearing the progressive power lens and it is determined that the subject requires expansion of the clear vision area in the horizontal direction, then, by selecting the horizontal expansion design, the progressive power lens that is worn more comfortably can be determined.

In this specification, the clear vision area is estimated from the addition power and the amount of astigmatism, but the optical transfer function (OTF) or modular transfer function (MTF) can also be used, or a value obtained by multiplying the OTF or MTF by the contrast sensitivity function (CSF) of the visual system as a weight and integrating the result over a certain range of spatial frequencies, can also be used. The above calculation may take into account a lens alone or physiological factors such as the aberration of the eye, the eye axis, the visual axis, etc. Also, the threshold value may reflect the degree of blur perception.

### Description of signs and numerals

10 Subject
20 Near target
30 Far target
40 Original image
40A, 40B Blurred images
S101 Clear vision area necessity determination step
S102 Design selection step

## Claims

1. A method for determining a progressive power lens, the method comprising:
determining a necessity for expanding a clear vision area for a subject wearing a progressive power lens; and when it is determined that the subject requires expansion of the clear vision area in a depth direction,
selecting a design of the progressive power lens in which the clear vision area is expanded in the depth direction by reducing an addition power in an intermediate portion of a progressive zone of the progressive power lens.

2. The method for determining a progressive power lens according claim 1, wherein in the determination step, the necessity for expanding the clear vision area is determined based on a transition of a line of sight movement of the subject when the subject alternates between near vision and far vision.

3. The method for determining a progressive power lens according to claim 1, wherein in the determination step, the necessity for expanding the clear vision area is determined based on an amount of third-order aberration in the horizontal direction of the subject's eye.

4. The method for determining a progressive power lens according to claim 1, wherein in the determination step, the necessity for expanding the clear vision area is determined based on a transition movement of an object when the subject performs a task including actions of holding, viewing, and moving the object whose position information can be acquired.

5. The method for determining a progressive power lens according to claim 1, wherein in the design selection step, when the subject's eye is an origin, a vertical direction is Y axis, and the depth direction is Z axis, a design with reduced addition power is selected so that a position of Y = - 210 mm, Z = 850 mm is within the clear vision area.

6. The method for determining a progressive power lens according to claim 1, wherein in the design selection step, a design of the progressive power lens is selected in which the clear vision area is expanded in the depth direction in exchange for increasing an amount of astigmatism on a principal meridian of the progressive power lens.

7. The method for determining a progressive power lens according to claim 1, wherein in the design selection step, by reducing the addition power, a design of a progressive power lens design is selected in which the clear vision area is expanded in the depth direction and horizontal direction.

8. The method for determining a progressive power lens according claim 1, wherein when it is determined that the subject requires expansion of the clear vision area in a horizontal direction in the determination step, a design of the progressive power lens is selected in the design selection step in which a power distribution of the progressive power lens is smoothed in the horizontal direction to reduce third-order aberration in the horizontal direction around a principal meridian of the progressive power lens, thereby expanding the clear vision area in the horizontal direction.

9. The method for determining a progressive power lens according to claim 8, wherein in the determination step, necessity for expanding the clear vision area is determined based on an amount of third-order aberration in the horizontal direction of the subject's eye.

10. The method for determining a progressive power lens according to claim 8, wherein a width of a smoothing filter for smoothing the power distribution in the horizontal direction is larger than a narrowest width among widths between peaks of third-order aberration on both left and right sides of a principal meridian in the progressive power lens before smoothing.

11. The method for determining a progressive power lens according to claim 8, wherein in the design selection step, a design of a progressive power lens in which the clear vision area is expanded in the horizontal direction in exchange for increasing the amount of astigmatism on the principal meridian of the progressive power lens, is selected.

12. The method for determining a progressive power lens according to claim 8, wherein in the design selection step, when it is determined that the subject requires expansion of the clear vision area in the horizontal direction, a design of a progressive power lens in which the clear vision area is expanded in the horizontal direction without changing an addition curve of the principal meridian, is selected.

13. The method for determining a progressive power lens according to any one of claims 1 to 12, wherein in the design selection step, when it is determined that the subject does not require expansion of the clear vision area, a basic design of the progressive power lens is selected in the design selection step.
